# FASCICULE DE BREVET EUROPEEN

(11) **EP 4 536 267 B1**
(45) Date de publication et mention de la délivrance du brevet: **29.04.2026**
(21) Numéro de dépôt: 23732052.8
(22) Date de dépôt: 09.06.2023
(51) Int. Cl.: A61K 38/17, A61K 9/00, A61P 27/02

(54) **HÉMOGLOBINE D'ANNÉLIDES POUR SON UTILISATION DANS LE TRAITEMENT DE LA MALADIE DE FUCHS**
ANNELIDHÄMOGLOBIN ZUR VERWENDUNG BEI DER BEHANDLUNG VON FUCHS-KRANKHEIT
ANNELID HEMOGLOBIN FOR USE IN THE TREATMENT OF FUCHS' DISEASE

(30) Priorité: 09.06.2022 FR 2205545
(43) Date de publication de la demande: 16.04.2025
(73) Titulaire: Hemarina, 29600 Morlaix (FR); Fondation A de Rothschild, 75019 Paris (FR)
(72) Inventeur: GABISON, Eric, 94200 Charenton le pont (FR); PERRAUD, Ludovic, 75012 PARIS (FR); SOUQUET, Benoit, 75010 PARIS (FR); GUINDOLET, Damien, 75018 PARIS (FR); ZAL, Franck, 29600 MORLAIX (FR)
(74) Mandataire: Lavoix
(86) Numéro de dépôt international: PCT/EP2023/065476
(87) Numéro de publication internationale: WO 2023/237733

(56) Documents cités:
- BATOOL FAREEHA ET AL: "Therapeutic Potential of Hemoglobin Derived from the Marine Worm Arenicola marina (M101): A Literature Review of a Breakthrough Innovation", MARINE DRUGS, vol. 19, no. 7, 1 July 2021 (2021-07-01), Basel, CH, pages 376, XP055860839, ISSN: 1660-3397, DOI: 10.3390/md19070376
- PRICE MARIANNE O ET AL: "Corneal endothelial dysfunction: Evolving understanding and treatment options", PROGRESS IN RETINAL AND EYE RESEARCH, OXFORD, GB, vol. 82, 22 September 2020 (2020-09-22), XP086618622, ISSN: 1350-9462, [retrieved on 20200922], DOI: 10.1016/J.PRETEYERES.2020.100904

## Description

La présente invention concerne l'utilisation d'au moins une molécule choisie parmi une globine d'Annélides, un protomère de globine d'Annélides et une hémoglobine extracellulaire d'Annélides, pour le traitement de la maladie de Fuchs.

L'endothélium cornéen, situé au niveau postérieur de la cornée, joue un rôle clé dans son maintien dans un état de déshydratation relative, essentiel au maintien la clarté de la cornée (1). L'endothélium cornéen est une monocouche cellulaire reposant sur la membrane de Descemet, sa membrane basale, sécrétée par les cellules endothéliales et qui s'épaissit tout au long de la vie. L'homéostasie de la matrice extracellulaire contenue dans la membrane de Descemet est régie par un équilibre entre la production et la lyse de matrice extra cellulaire par le couple des métalloprotéinases (MMPs) et de leurs inhibiteurs (TIMP) (2). Chez l'humain, la densité cellulaire endothéliale est de 4000 cellules/mm² à la naissance et diminue progressivement tout au long de la vie (3). Une perte excessive de cellules endothéliales peut être la conséquence de multiples mécanismes pathologiques, tels qu'une infection (telle qu'une endothélite virale), un traumatisme (par exemple après chirurgie de la cataracte) ou encore de pathologies génétiques. Au-delà d'un certain seuil de perte en cellules endothéliales, s'installe un état pathologique appelé « insuffisance endothéliale », entraînant une accumulation d'eau dans le stroma cornéen ou oedème cornéen, responsable d'une perte de la transparence cornéenne et d'une baisse de l'acuité visuelle.

Une des principales causes d'insuffisance endothéliale est la dystrophie endothéliale cornéenne de Fuchs, qui est la première indication de greffe de cornée dans le monde (4). C'est une maladie plurifactorielle entraînant des anomalies cellulaires et une mort cellulaire accrue résultant *in fine* en une baisse progressive de la densité en cellules endothéliales. Sa physiopathologie fait intervenir un stress du réticulum endoplasmique avec formation d'aggrésomes d'une part (5), et une dysfonction des métalloprotéinases d'autre part (6).

Cette maladie se caractérise notamment par la présence de gouttes dans les régions les plus sévèrement atteintes (au centre de la cornée). Les gouttes correspondent à un dépôt anormal de matrice extra cellulaire au niveau de la membrane de Descemet. Elles peuvent être observées cliniquement chez 9 à 11% des femmes contre 3,5 à 9% des hommes (7).

Seule une faible proportion des patients atteints de dystrophie de Fuchs nécessite une greffe de la cornée. Ces patients rapportent, au stade initial, un flou visuel transitoire le matin au réveil correspondant cliniquement à un œdème de cornée transitoire. Cet œdème de cornée pourrait être dû à une hypoxie cornéenne survenant au niveau d'un endothélium pathologique au cours d'une fermeture palpébrale prolongée. Les données de la littérature font part d'un dysfonctionnement endothélial lors du port prolongé de lentilles de contact ayant pour conséquence clinique un œdème de cornée transitoire. Ce dysfonctionnement endothélial est la conséquence d'une hypoxie cornéenne prolongée (8).

Les traitements de la dystrophie endothéliale cornéenne de Fuchs sont aujourd'hui limités ; ils sont d'ordre médical et d'ordre chirurgical (voir par exemple Price et al., Progress In Retinal and Eye Research, vol. 82, 22 septembre 2020).

D'une part, certains collyres sont utilisés pour diminuer l'œdème cornéen. Cependant, leur efficacité variable et toujours limitée aux stades précoces de la maladie et ne constituent qu'un traitement palliatif de l'atteinte cornéenne.

D'autre part, le traitement de référence reste la greffe de la cornée. Plus précisément, la greffe est effectuée par une transposition d'un feuillet de cellules de l'endothélium et de Descemet. Cette technique, appelée DMEK (Descemet Membrane Endothelial Keratoplasty) a pour avantage de ne remplacer que le tissu malade. Cependant, cette technique reste invasive et transitoire, puisque la durée de vie du greffon est estimée à une dizaine d'année en absence de rejet.

Il existe donc un besoin pour un traitement efficace de la dystrophie endothéliale cornéenne de Fuchs, qui soit facile à administrer et non invasif. Un tel traitement permettrait notamment de pallier le manque de greffon et d'éviter la greffe de cornée.

La présente invention répond à ce besoin.

De manière surprenante, comme décrit dans les exemples, les inventeurs ont montré que l'hypoxie intermittente participe à l'évolution de la pathologie en aggravant le stress du réticulum endoplasmique et en modifiant l'expression de facteurs impliqués dans la physiopathologie de la maladie comme BAG3 (BCL2-Associated Athanogene 3) et MMP2 (métalloprotéinase de type 2). Les inventeurs ont en effet identifié sur des échantillons de patients atteints par la maladie que BAG3, une protéine chaperone impliquée dans la formation d'aggrésomes en réponse à un stress cellulaire, interagit avec MMP2 et forme des agrégats au niveau des zones pathologiques caractérisées par la présence de gouttes. Ces formations résultent d'une rétention intracellulaire de MMP2 secondaire à un stress du réticulum endoplasmique.

Par ailleurs, certains modèles *in vitro* de dystrophie de Fuchs ont été décrits à ce jour, mais ils ont pour principal inconvénient de ne cibler qu'un aspect de la physiopathologie. Là encore, de manière surprenante, les inventeurs ont développé un modèle cellulaire *in vitro* de dystrophie de Fuchs à base de chlorure de cobalt, et un modèle d'hypoxie/réoxygénation mimant les variations d'oxygène rencontrées *in vivo.* Ce modèle cellulaire de dystrophie de Fuchs regrouperait en une seule molécule, le chlorure de cobalt, plusieurs caractéristiques physiopathologiques de la dystrophie de Fuchs.

Les inventeurs ont également découvert que l'utilisation de l'hémoglobine extracellulaire *d'Arenicola marina* permet de limiter les fluctuations nocturnes en oxygène au niveau de l'endothélium cornéen.

La présente invention se rapporte ainsi à l'utilisation d'au moins une molécule choisie parmi une globine d'Annélides, un protomère de globine d'Annélides et une hémoglobine extracellulaire d'Annélides, pour le traitement de la dystrophie endothéliale cornéenne de Fuchs.

L'utilisation selon l'invention comprend au moins une molécule choisie parmi une globine d'Annélides, un protomère de globine d'Annélides et une hémoglobine extracellulaire d'Annélides.

Cette molécule est un transporteur d'oxygène. Par « transporteur d'oxygène », on entend une molécule capable de transporter l'oxygène, de façon réversible, depuis l'environnement jusqu'aux cellules, tissus ou organes cibles.

L'hémoglobine extracellulaire d'Annélides est présente chez les trois classes d'Annélides: les Polychètes, les Oligochètes et les Achètes. On parle d'hémoglobine extracellulaire car elle est naturellement non contenue dans une cellule, et peut donc circuler librement dans le système sanguin sans modification chimique pour la stabiliser ou la rendre fonctionnelle.

L'hémoglobine extracellulaire d'Annélides est un biopolymère géant de poids moléculaire compris entre 2000 et 4000 kDa, constitué d'environ 200 chaînes polypeptidiques comprises entre 4 et 12 types différents que l'on regroupe généralement en deux catégories.

La première catégorie, comptant 144 à 192 éléments, regroupe les chaînes polypeptidiques dites "fonctionnelles" qui portent un site actif de type hème, et sont capables de lier réversiblement l'oxygène ; ce sont des chaînes de type globine (huit types au total pour l'hémoglobine d'*Arenicola marina :* a1, a2, b1, b2, b3, c, d1 et d2), dont les masses sont comprises entre 15 et 18 kDa. Elles sont très similaires aux chaînes de type α et β de vertébrés.

La deuxième catégorie, comptant 36 à 42 éléments, regroupe les chaînes polypeptidiques dites de « structure » ou « linkers » possédant peu ou pas de site actif mais permettant l'assemblage des sous-unités appelées douzièmes ou protomères. On compte deux types de linkers, L1 et L2.

Chaque molécule d'hémoglobine est constituée de deux hexagones superposés que l'on a nommés bicouche hexagonale (hexagonal bilayer) et chaque hexagone est lui-même formé par l'assemblage de six sous-unités (dodécamère ou protomère) en forme de goutte d'eau. La molécule native est formée de douze de ces sous-unités (dodécamère ou protomère). Chaque sous-unité a une masse moléculaire d'environ 250 kDa, et constitue l'unité fonctionnelle de la molécule native.

De préférence, l'hémoglobine extracellulaire d'Annélides est choisie parmi les hémoglobines extracellulaires d'Annélides Polychètes et les hémoglobines extracellulaires d'Annélides Oligochètes. De préférence, l'hémoglobine extracellulaire d'Annélides est choisie parmi les hémoglobines extracellulaires de la famille des *Lumbricidae,* les hémoglobines extracellulaires de la famille des *Arenicolidae* et les hémoglobines extracellulaires de la famille des *Nereididae.* Encore plus préférentiellement, l'hémoglobine extracellulaire d'Annélides est choisie parmi l'hémoglobine extracellulaire de *Lumbricus terrestris,* l'hémoglobine extracellulaire d'*Arenicola sp* et l'hémoglobine extracellulaire de *Nereis sp.* Plus préférentiellement selon l'invention, l'hémoglobine extracellulaire d'Annélides est choisie parmi l'hémoglobine extracellulaire *d'Arenicola marina* ou de *Nereis virens,* plus préférentiellement est l'hémoglobine extracellulaire *d'Arenicola marina.* L'arénicole *Arenicola marina* est un ver annélide polychète vivant essentiellement dans le sable.

Selon l'invention, le protomère de globine de l'hémoglobine extracellulaire d'Annélides constitue l'unité fonctionnelle de l'hémoglobine native, comme indiqué ci-dessus.

Enfin, la chaîne de globine de l'hémoglobine extracellulaire d'Annélides peut notamment être choisie parmi les chaînes de globine de type Ax et/ou Bx d'hémoglobine extracellulaire d'Annélides.

L'hémoglobine extracellulaire d'Annélides, ses protomères de globine et/ou ses globines ne nécessitent pas de cofacteur pour fonctionner, contrairement à l'hémoglobine de mammifère, notamment humaine. Enfin, l'hémoglobine extracellulaire d'Annélides, ses protomères de globine et/ou ses globines ne possédant pas de typage sanguin, ils permettent d'éviter tout problème de réaction immunologique ou allergique. L'hémoglobine extracellulaire d'Annélides, ses protomères de globine et/ou ses globines présentent une activité superoxide dismutase (SOD) intrinsèque. Par conséquent, cette activité anti-oxydante intrinsèque ne nécessite aucun antioxydant pour fonctionner, contrairement à l'utilisation d'une hémoglobine de mammifères pour laquelle les molécules antioxydantes sont contenues à l'intérieur du globule rouge et ne sont pas liées à l'hémoglobine.

L'hémoglobine extracellulaire d'Annélides, ses protomères de globine et/ou ses globines peuvent être natifs ou recombinants.

De préférence, l'hémoglobine extracellulaire est celle *d'Arenicola marina* ou celle de *Nereis virens,* plus préférentiellement l'hémoglobine extracellulaire *d'Arenicola marina.*

De préférence, la molécule est présente dans une composition en une teneur comprise entre 0,01% et 10% en poids par rapport au poids total de composition, de préférence entre 0,05% et 5% en poids, de préférence entre 0,06% et 2% en poids, de préférence entre 0,07% et 1% en poids.

De préférence, l'utilisation selon l'invention permet de réduire significativement l'hypoxie intermittente des cellules endothéliales de la cornée, donc de réduire le stress du réticulum endoplasmique des cellules endothéliales de la cornée.

Par « réduire significativement l'hypoxie intermittente des cellules endothéliales de la cornée », on entend diminuer significativement le stress cellulaire induit par l'hypoxie nocturne survenant au niveau de l'endothélium pathologique. Ce phénomène survient la nuit, au cours de la fermeture palpébrale prolongée. La réduction de l'hypoxie intermittente des cellules endothéliales de la cornée peut être mesurée grâce au modèle d'hypoxie décrit en exemple, notamment par la mesure des marqueurs *MMP2* et/ou *BAG3.*

De préférence, l'utilisation selon l'invention permet de réduire significativement l'expression de *MMP2* et/ou de *BAG3* dans l'endothélium cornéen.

Par « réduire significativement l'expression de *MMP2* et/ou de *BAG3* dans l'endothélium cornéen », on entend une réduction de la transcription et/ou de la traduction de *MMP2* et/ou de *BAG3* dans les cellules endothéliales cornéennes, d'au moins 20%, de préférence d'au moins 30%, de préférence d'au moins 40%, de préférence d'au moins 50%. De préférence, l'utilisation d'au moins une molécule choisie parmi une globine d'Annélides, un protomère de globine d'Annélides et une hémoglobine extracellulaire d'Annélides selon l'invention réduit la transcription et/ou la traduction de *MMP2* et/ou de *BAG3* dans les cellules endothéliales cornéennes, d'au moins 20%, de préférence d'au moins 30%, de préférence d'au moins 40%, de préférence d'au moins 50%, par rapport à des cellules endothéliales cornéennes témoins. Les témoins peuvent être des cellules endothéliales cornéennes non traitées par la molécule, ou bien les cellules endothéliales cornéennes avant traitement avec la molécule. La transcription et/ou de la traduction de *MMP2* et/ou de *BAG3* peuvent être mesurées par toute méthode connue de l'art antérieur, et notamment tel que décrit en exemple.

De préférence, l'utilisation selon l'invention permet de protéger les cellules endothéliales cornéennes du stress d'hypoxie/réoxygénation.

Par « protéger les cellules endothéliales cornéennes du stress hypoxique », on entend une diminution de l'expression d'au moins un marqueur de stress du réticulum endoplasmique des cellules endothéliales cornéennes, et/ou une diminution de l'expression d'au moins un marqueur d'autophagie des cellules endothéliales cornéennes, de préférence BAG3, et/ou une augmentation de la viabilité cellulaire des cellules endothéliales cornéennes, d'au moins 20%, de préférence d'au moins 30%. De préférence, le marqueur de stress du réticulum endoplasmique est choisi parmi *HSPA5* (BIP), DDIT3 (CHOP) et *sXBP1.* De préférence, le marqueur d'autophagie des cellules endothéliales cornéennes est BAG3. De préférence, l'utilisation d'au moins une molécule choisie parmi une globine d'Annélides, un protomère de globine d'Annélides et une hémoglobine extracellulaire d'Annélides selon l'invention diminue l'expression d'au moins un marqueur de stress du réticulum endoplasmique des cellules endothéliales cornéennes, et/ou diminue l'expression d'au moins un marqueur d'autophagie des cellules endothéliales cornéennes, de préférence BAG3, et/ou augmente la viabilité cellulaire des cellules endothéliales cornéennes, d'au moins 20%, de préférence d'au moins 30%, par rapport à des cellules endothéliales cornéennes témoins. Les témoins peuvent être des cellules endothéliales cornéennes non traitées par la molécule, ou bien les cellules endothéliales cornéennes avant traitement avec la molécule. L'expression d'au moins un marqueur de stress du réticulum endoplasmique des cellules endothéliales cornéennes, l'expression d'au moins un marqueur d'autophagie des cellules endothéliales cornéennes et la viabilité cellulaire des cellules endothéliales cornéennes, peuvent être mesurées selon toute méthode connue de l'art antérieur, et notamment tel que décrit en exemple.

De préférence, la molécule choisie parmi une globine d'Annélides, un protomère de globine d'Annélides et une hémoglobine extracellulaire d'Annélides selon l'invention, est formulée dans une solution tampon. La solution obtenue (i.e. solution tampon comprenant la molécule) peut être lyophilisée, afin d'obtenir une poudre. De préférence, la solution obtenue (i.e. solution tampon comprenant la molécule) est utilisée telle quelle (forme liquide), sous forme non lyophilisée.

Typiquement, la solution tampon crée un environnement salin adéquat pour l'hémoglobine, ses protomères et ses globines, et permet ainsi le maintien de la structure quaternaire, et donc de la fonctionnalité de cette molécule. La solution tampon est de préférence une solution aqueuse comprenant des sels, de préférence des ions chlorure, sodium, calcium, magnésium et potassium, et son pH est compris entre 5 et 9, de préférence entre 5.5 et 8.5, idéalement entre 7.4 et 7.7 (ce qui correspond au pH physiologique du film lacrymal). Sa formulation est similaire à celle d'un liquide physiologiquement injectable. De préférence, la solution tampon comprend également un antioxydant, tel que l'acide ascorbique. Dans ces conditions, l'hémoglobine extracellulaire d'Annélides, ses protomères de globine et ses globines restent fonctionnels.

Dans la présente description, le pH s'entend à température ambiante (25°C), sauf mention contraire. De préférence, la solution tampon est une solution aqueuse comprenant du chlorure de sodium, du chlorure de calcium, du chlorure de magnésium, du chlorure de potassium, ainsi que du sodium gluconate et du sodium acétate, et a un pH compris entre 6.5 et 7.6, de préférence égal à 7,1 ± 0,5, de préférence d'environ 7.35. Plus préférentiellement, la solution tampon est une solution aqueuse comprenant 90 mM de NaCl, 23 Mm de Na-gluconate, 2,5 mM de CaCl₂, 27 mM de Na-acétate, 1,5 mM de MgCl₂, 5 mM de KCI, et a un pH de 7,1 ± 0,5. De préférence, la solution tampon présente une osmolarité proche de celle du film lacrymal, i.e. comprise entre 270 et 315 mOsm/L, de préférence d'environ 300 mOsm/L.

De préférence, la molécule choisie parmi une globine d'Annélides, un protomère de globine d'Annélides et une hémoglobine extracellulaire d'Annélides selon l'invention (formulée ou non dans une solution tampon) est formulée dans une composition pharmaceutique de préférence convenant pour une administration oculaire.

Par « composition pharmaceutique convenant pour une administration oculaire », on entend toute composition pharmaceutique (médicament) ayant une forme galénique convenant pour une administration oculaire.

De préférence, la composition pharmaceutique convenant pour une administration oculaire est choisie parmi les collyres, les pommades ophtalmiques, les gels ophtalmiques, les inserts conjonctivaux et les lentilles thérapeutiques.

Les collyres sont des préparations liquides (par exemple des solutions, des suspensions ou des émulsions) stériles destinées au traitement des affections de l'oeil. Ils sont typiquement présentés dans des flacons spécifiques multidoses de 5 à 10 ml avec un embout compte-gouttes ou en doses unitaires (ophtadoses). Le conditionnement est typiquement adapté à l'administration du collyre, et peut prévoir un volume maximal d'une goutte d'environ 30µl. Les collyres comprennent généralement un solvant, de préférence aqueux. La formulation peut comprendre un adjuvant, de préférence l'acide borique ou l'un de ses sels, et/ou des agents isotonisants tels que chlorure de sodium, et/ou de la vitamine C ou ses dérivés, de préférence l'acide ascorbique. Les collyres peuvent comprendre un tensioactif, de préférence choisi parmi les polysorbates, les polyoxyéthylènes et le tyloxapol. Le tensioactif permet d'améliorer la solubilité du principe actif. Les collyres sont stériles et isotoniques (pH compris entre 6.4 et 7.8).

Les pommades ophtalmiques présentent une consistance semi-solide. Elles sont utilisées pour avoir un effet plus prolongé car le principe actif est maintenu au contact de l'œil plus longtemps. Elles sont également prescrites dans le traitement des affections des paupières (blépharites ou orgelets). Parmi les excipients couramment rencontrés dans les pommades ophtalmiques, on peut citer de préférence la vaseline ou la paraffine liquide.

Les gels ophtalmiques sont des préparations semi-solides stériles destinées à être appliquées sur la conjonctive. Généralement elles contiennent un ou plusieurs principe(s) actif(s) dissous dans un excipient approprié. L'excipient est typiquement un polymère hydrophile qui gélifie en présence d'eau, par exemple un carbomère, un carbopol ou un acide polyacrylique.

Les inserts conjonctivaux sont des dispositifs implantés sous la paupière. Un exemple de ce type d'insert, à base de tropicamide et de chlorhydrate de phényléphrine, est la référence Mydriasert.

Les lentilles thérapeutiques sont des dispositifs médicaux qui maintiennent la vision binoculaire (par exemple par rapport à un pansement oculaire). Une telle lentille peut être pré-imprégnée par la molécule choisie parmi une globine d'Annélides, un protomère de globine d'Annélides et une hémoglobine extracellulaire d'Annélides selon l'invention, formulée ou non dans une solution tampon.

L'invention a également pour objet un modèle *in vitro* de dystrophie de Fuchs, comprenant des cellules, de préférence des cellules saines d'endothélium cornéen, et un milieu de culture comprenant du chlorure de cobalt (CoCl2). Par « cellules saines d'endothélium cornéen », on entend des cellules non atteintes de dystrophie de Fuchs. De préférence, CoCl2 est présent en une concentration allant de 1 à 15 µM, de préférence de 2µM à 10µM. De préférence, les cellules saines d'endothélium cornéen sont la lignée endothéliale HCEC B4G12.

Le chlorure de cobalt (CoCl2) peut être utilisé comme additif dans un milieu de culture cellulaire ; lorsque ledit milieu de culture est avec des cellules saines d'endothélium cornéen, on peut obtenir un modèle *in vitro* de dystrophie de Fuchs.

L'invention est illustrée par les exemples et les figures suivants.

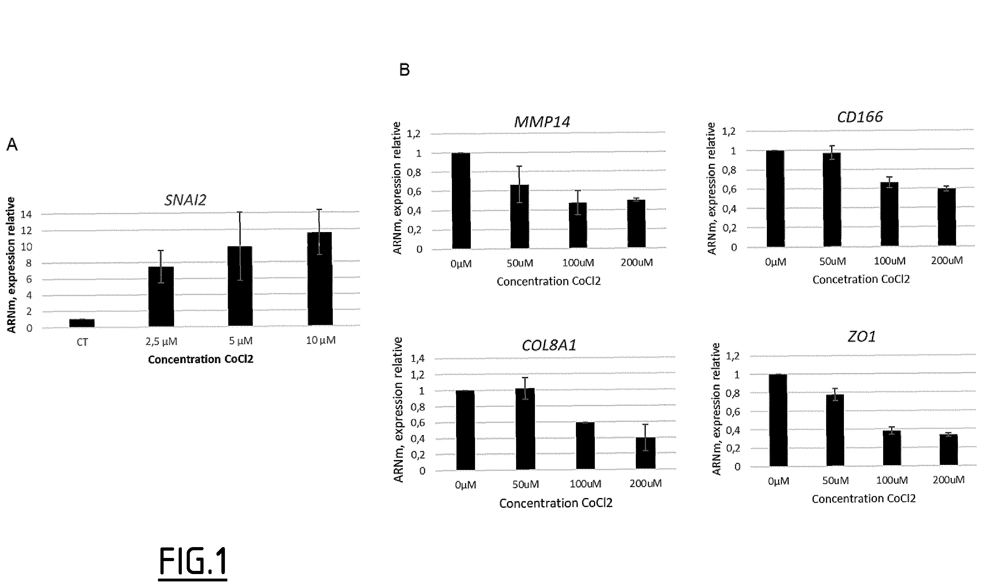

[Fig 1] Le chlorure de cobalt induit une perte du phénotype endothélial avec transition endothélio-mésenchymateuse sur la lignée endothéliale HCEC B4G12.
   A : Expression génique, marqueur de transition endothélio-mésenchymateuse *SNAI2*, culture prolongée. Un traitement prolongé pendant 7 jours avec du chlorure de cobalt induit l'expression du gène de transition endothélio-mésenchymateuse *SNAI2* de manière dose dépendante ;
   B : Expression génique, marqueurs de différentiation endothéliale *ZO1, COL8A1, MMP14* et *CD166,* culture courte. Un traitement en cure courte (24h) entraîne une diminution d'expression des marqueurs de différentiation endothéliale *ZO1, COL8A1, MMP14* et *CD166,* de manière dose dépendante.
[Fig 2] Induction de l'expression génique des marqueurs de stress du réticulum endoplasmique par le chlorure de cobalt (CoCl2).
[Fig 3] Induction de l'expression génique de *BAG3* et *MMP2* par l'hypoxie/réoxygénation (H/R) avec ou sans chlorure de cobalt (CoCl2).
[Fig 4] L'hémoglobine *d'Arenicola marina* (M101) à 0,5g/l diminue l'effet de l'hypoxie/réoxygénation (H/R) et du chlorure de cobalt (CoCl2) sur l'expression génique de *BAG3* et *MMP2.*

### Exemple

### Matériels et Méthodes :

### Culture cellulaire :

HCEC-B4G12 (DSZM^{®}, Braunschweig, Allemagne) est une sous-population clonale créée à partir de la lignée cellulaire parentale HCEC-12 (ACC 646) établie à partir de cellules normales d'endothélium cornéen d'une femme caucasienne de 91 ans transformée avec la région du génome SV40.

Les flasques doivent être coatées avec une solution contenant 10 µg/mL de laminine (L2020, Sigma^{®}, Saint-Louis, Etats-Unis), et de 10 mg/mL de chondroïtine sulfate (C4383, Sigma^{®}, Saint-Louis, Etats-Unis). Le milieu de culture est composé de Human Endothelial SFM (11111-044 Gibco^{®}, Carlsbad, États-Unis) avec 10ng/mL FGF2 (233-FB-025 Sigma^{®}, Saint-Louis, Etats-Unis), les cellules sont cultivées à 37°C avec 5 % de CO2. Le milieu de culture est changé toutes les 48 heures. Les cellules atteignent la confluence en une semaine. Les cellules sont passées à confluence après incubation avec de la Trypsine TripleExpress (2604-021 Gibco^{®}, Carlsbad, États-Unis) pendant 5 minutes à 37°C.

### Modèle in vitro de dystrophie de Fuchs :

Le chlorure de cobalt (CoCl2) (C8661, Sigma^{®}, Saint-Louis, Etats-Unis) est utilisé comme inducteur d'un trouble de la protéostase. Une solution stock à 25µM est préparée en diluant 12mg de chlorure de cobalt sous forme de poudre dans 2ml d'eau distillée stérile et conservée à 4°C pendant une semaine. Le chlorure de cobalt est ensuite dilué directement dans le milieu de culture endothélial (Human Endothelial SFM + FGF2) pour obtenir les concentrations voulues : 2,5µM, 5µM ou 10µM. Les milieux sont changés toutes les 48 heures en présence ou non du chlorure de cobalt avec un temps de culture total de 8 jours.

### Test de sénescence cellulaire :

L'activité béta-galactosidase des cellules est détectée grâce au kit de coloration histochimique des cellules sénescentes (CS0030, Sigma^{®}, Saint-Louis, Etats-Unis) selon les recommandations du fournisseur. Les cellules HCEC B4G12 sont cultivées pendant une semaine avec ou sans chlorure de cobalt (concentration allant de 2,5 à 30µM) dans une plaque 24 puits puis fixées à l'aide du tampon de fixation du kit pendant 7 minutes à température ambiante. Après rinçage au PBS, les cellules sont incubées avec la solution de coloration contenant le X-gal toute la nuit à 37°C sans CO2. Après incubation, les cellules sont observées sous microscope, le cytoplasme des cellules sénescentes apparaît bleu.

### Modèle d'hypoxie :

Un incubateur à hypoxie (PHCBI^{®}, Bernolsheim, France) a été utilisé. Dans un premier temps, les cellules sont cultivées jusqu'à confluence en normoxie, soit environ 7 jours après ensemencement, puis transférée en milieu hypoxique à 1% O2, 5% CO2, 94% NO à 37°C pendant 12 heures. Un cycle d'hypoxie/réoxygénation (H/R) est ensuite réalisé en incubant les cellules de nouveau en normoxie 21% d'O2, 5 % de CO2 à 37 °C après changement du milieu. Pour une analyse de l'expression génique en RT-qPCR le temps de réoxygénation est de 1h, pour analyse l'expression protéique en immunofluorescence le temps de réoxygénation est de 3h.

### Utilisation de M101 dans le modèle in vitro :

L'hémoglobine *d'Arenicola marina* (M101) selon l'invention est délivrée par la société Hemarina, Morlaix, France. Une solution stock à 50g/l est conservée à -80°C. La solution est décongelée à température ambiante avant utilisation et est directement diluée dans le milieu de culture avant l'épreuve d'hypoxie/réoxygénation à une concentration allant de 1g/L à 0,1g/L. Tous les contrôles sont effectués en présence de la solution de conservation aux mêmes volumes que les conditions traitées.

### RT-_{Q}PCR :

L'ARN est extrait avec le kit 'RNAeasy Mini Plus' 74136 (Qiagen^{®}, Hilden, Allemagne) selon les instructions du constructeur. L'ARN ainsi isolé était quantifié à l'aide de la plateforme de détection Tecan^{®} (Mannedorf, Suisse) en réalisant un dosage spectrophotométrique. On obtient ensuite l'ADNc de chaque échantillon par transcription inverse en utilisant la SuperScript III (ThermoFischer^{®}, Waltham, Etats-Unis) selon les instructions du fabricant. Chaque puits contient 20µL de solution avec 1µg d'ARN, 10µL de RT Reaction Mix, 2µL d'enzyme et un complément variable d'eau. La PCR quantitative est ensuite réalisée pour chaque échantillon d'ADNc amplifié à l'aide de l'appareil à qPCR QuantStudio5 (ThermoFischer^{®}, Waltham, Etats-Unis) en utilisant la méthode de détection SYBR-Green. Les courbes sont analysées par le logiciel QuantStudio5 software. Les amorces utilisées pour les différents gènes d'intérêt sont décrites dans le tableau 1. Le gène de ménage, Béta-2 Microglobuline, utilisé a été sélectionné conformément aux données de la littérature concernant la lignée. En parallèle, différents gènes de ménage connus de la littérature *(B2M, GAPDH, PPIA*) ont été testés sur différents échantillons de la lignée. *B2M* s'est avéré être le gène le moins susceptible aux variations de conditions expérimentales avec un seuil de détection constant sur les différents échantillons.

**[Table 1]**

| **Gènes d'intérêt** | **Amorce Forward** | **Amorce Reverse** |
|---|---|---|
| *BAG3* | GGAGATCAAGATCGACCCGC (SEQ ID NO :1) | AGAGGATGGAGTCTCCTTGGG (SEQ ID NO :2) |
| *B2M* | ACTGAATTCACCCCCACTGA (SEQ ID NO :3) | CCTCCATGATGCTGCTTACA (SEQ ID NO :4) |
| *CD166* | CCCCAGAGGAATTTTTGTTTTAC (SEQ ID NO :5) | AGCCTGATGTTATCTTTCATCCA (SEQ ID NO:6) |
| *COL8A1* | CCAACTCACCCTTGAAGTCAT (SEQ ID NO :7) | GGCTGGTTTCTGTCTCTTCAG (SEQ ID NO :8) |
| *DDIT3* | ATGGCAGCTGAGTCATTGCCTTTC (SEQ ID NO :9) | AGAAGCAGGGTCAAGAGTGGTGAA (SEQ ID NO :10) |
| *HSPA5* | CGAGGAGGAGACAAGAAGG (SEQ ID NO:11) | CACCTTGAACGGCAAGAACT (SEQ ID NO :12) |
| *MMP2* | GTGAAGTATGGGAACGCCGA (SEQ ID NO :13) | AGAAGCCGTACTTGCCATCC (SEQ ID NO :14) |
| *MMP14* | TCCAGCAACTTTATGGGGGT (SEQ ID NO :15) | TTCCCGTCACAGATGTTGGG (SEQ ID NO :16) |
| *SNAI2* | TGGTTGCTTCAAGGACACAT (SEQ ID NO :17) | GCAAATGCTCTGTTGCAGTG (SEQ ID NO :18) |
| *sXBP1* | CTGAGTCCGAATCAGGTGCA (SEQ ID NO :19) | ATCCATGGGGAGATGTTCTGG (SEQ ID NO :20) |
| *ZO1* | GGTCAGAGCCTTCTGATCATTC (SEQ ID NO :21) | CATCTCTACTCCGGAGACTGC (SEQ ID NO :22) |

### Immunofluorescence sur lignée cellulaire HCEC B4G12 :

Les cellules sur lamelles sont fixées avec du PFA (1040051000 Merck^{®}, Darmstadt, Allemagne) dilué à 4% pendant 15min. Les lamelles sont incubées avec le tampon de perméabilisation et de saturation : 0,5% de Tween 20 (85113 ThermoScientific^{®}, Waltham, Etats-Unis), 1% de Triton (437002A VWR^{®} Radnor, Etats-Unis), 5% d'albumine Fraction V (1.12018.0100 Merck^{®}, Darmstadt, Allemagne) pendant 1h à température ambiante puis incubées avec les anticorps primaires pendant 3h dans le tampon de saturation. La dilution des anticorps primaires dans le tampon de saturation est la suivante : BAG3 (Rabbit 10599-1-AP Proteintech^{®}) (1/100), MMP2 Rabbit (37150 Abcam^{®}) (1/200), MMP2 Mouse (436000 Invitrogen^{®}) (1/100), alpha-tubuline (MAB1864, Merck^{®}, Darmstadt, Allemagne) (1/400).

Les lamelles sont ensuite rincées avec du PBS 5min (x3), puis incubées avec le mélange d'anticorps secondaires et de DAPI pendant une heure à température ambiante à l'abri de la lumière dans le tampon de perméabilisation et saturation. La dilution des anticorps secondaires est la suivante : Alexa Fluor 555 goat anti-rabbit (A21428 ThermoScientific^{®}, Waltham, Etats-Unis) (1/500) et Alexa Fluor 488 goat anti-mouse (A11001 Thermo ThermoScientific^{®}, Waltham, Etats-Unis) (1/500) avec du DAPI (62248 ThermoScientific^{®}, Waltham, Etats-Unis) (1/2000) dilués dans le tampon de saturation. Les lamelles sont rincées au PBS 5min (x3), puis montées sur lames avec un milieu de montage Permafluor (TA-030-FM TermoScientifc^{®}, Waltham, Etats-Unis). Tous les contrôles négatifs sont effectués en l'absence d'anticorps primaire mais en présence d'anticorps secondaires. L'acquisition des images est réalisée à l'aide du microscope confocal LSM 800 (Zeiss^{®}, Oberkochen, Allemagne). Les images sont ensuite analysées avec le logiciel Fiji^{®}. Chaque anticorps primaire est testé au moins trois fois dans les différentes conditions expérimentales.

### Résultats

### Données préliminaires sur des échantillons de patients (Images non montrées)

En immunofluorescence, une augmentation du signal du marqueur du stress du réticulum endoplasmique BIP (ou GRP78) a été retrouvée sur des échantillons de patients, ce qui correspond aux données actuelles de la littérature plaçant le stress du réticulum au centre de la physiopathologie de la dystrophie de Fuchs.

Sur des reconstitutions en projection orthogonale, les inventeurs ont pu mettre en évidence une perte de la distribution linéaire de MMP2 au sein de la membrane de Descemet en comparaison à des sujets sains. Sur des endothélio-Descemet pathologiques, MMP2 forme des aggrégats intracellulaires au niveau du domaine périnucléaire.

On met en évidence également une modification de l'expression concernant BAG3. Son expression est cytoplasmique et diffuse en immunofluorescence chez un sujet sain. Chez un sujet atteint de dystrophie de Fuchs, on observe des modifications régionales de l'expression de BAG3. On retrouve BAG3 dans le domaine périnucléaire au niveau des cellules situées en périphérie d'une endothélio-Descemet pathologique (dans les zones sans goutte), alors que dans la zone centrale de l'endothélio-Descemet riche en gouttes, le signal de BAG3 est nucléaire et forme des aggrégats cytoplasmiques périnucléaires co-localisant avec MMP2.

Un co-marquage MMP2 et LCIII, marqueurs d'aggresomes, sur des échantillons de patients indique la présence d'aggrésomes contenant MMP2.

Les données préliminaires des analyses d'échantillons de patients suggèrent que BAG3 est impliqué dans la formation d'aggresomes sur des endothélio-Descemet pathologiques dans lesquelles se trouve MMP2.

### Le chlorure de cobalt induit une perte du phénotype endothélial avec transition endothélio-mésenchymateuse sur la lignée endothéliale HCEC B4G12

Lors d'un traitement prolongé avec le chlorure de cobalt dans le milieu de culture pour des doses allant de 2,5µM à 10µM, les cellules perdent leur phénotype endothélial avec notamment une perte de leur aspect jointif et hexagonal (photos non montrées) et la présence de cellules d'allure fibroblastique au cytoplasme plus gros et allongé.

De la même manière, un traitement prolongé pendant 7 jours avec du chlorure de cobalt induit l'expression du gène de transition endothélio-mésenchymateuse *SNAI2* de manière dose dépendante (Figure 1A).

L'activité béta-galactosidase, illustrée par la coloration bleue cytoplasmique périnucléaire (photos non montrées) des cellules endothéliales en présence de chlorure de cobalt, est également augmentée, témoignant du phénomène de sénescence cellulaire induit par la molécule.

Un traitement en cure courte (24h) entraîne une diminution d'expression des marqueurs de différentiation endothéliale *ZO1, COL8A1, MMP14* et *CD166,* de manière dose dépendante (Figure 1B).

Ces données suggèrent que le chlorure de cobalt entraîne une perte de différentiation endothéliale avec transition endothélio-mésenchymateuse, et induit une senescence cellulaire, phénomènes observés *in vivo* chez les patients atteints de la dystrophie de Fuchs.

### Le chlorure de cobalt induit un stress du réticulum endoplasmique sur la lignée endothéliale HCEC B4G12

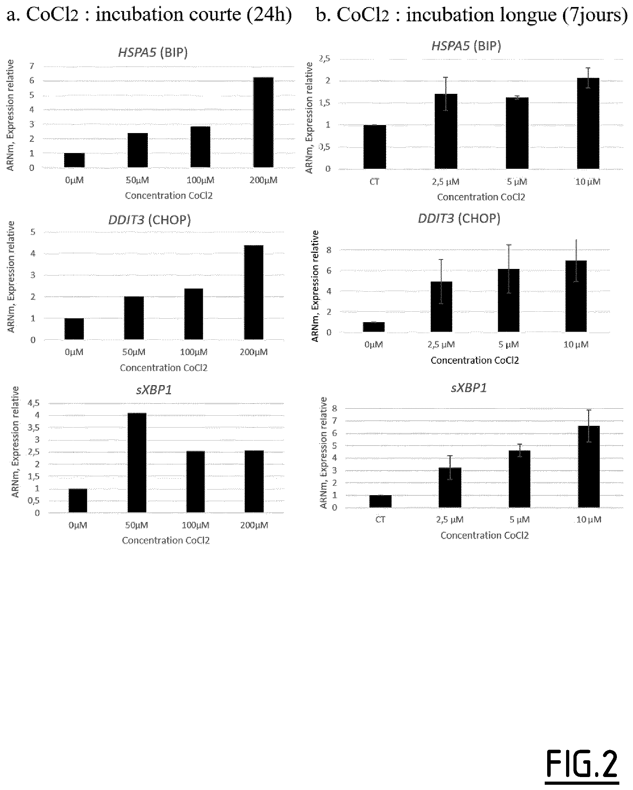

Le chlorure de cobalt induit une expression de marqueurs du réticulum endoplasmique, *HSPA5* (BIP), DDIT3 (CHOP) et *sXBP1* (Figure 2), évaluée par RT-qPCR, que ce soit lors d'une incubation courte de 24h (Figure 2a) pour des doses allant de 50 à 200µM, ou lors d'une culture prolongée de 7 jours (Figure 2b) pour des doses allant de 2,5 à 10µM.

En immunofluorescence, le chlorure de cobalt en culture prolongée induit la formation d'agrégats de MMP2 au niveau du domaine périnucléaire et le long des microtubules. On met également en évidence une augmentation du signal de BIP au niveau du réticulum endoplasmique en présence de chlorure de cobalt (Images non montrées).

L'utilisation du chlorure de cobalt permet de mimer *in vitro* le stress du réticulum endoplasmique et la formation d'aggrésomes tels qu'observés *in vivo* sur des échantillons de patients.

### L'hypoxie/réoxygénation modifie l'expression de BAG3 et MMP2 sur la lignée HCEC B4G12

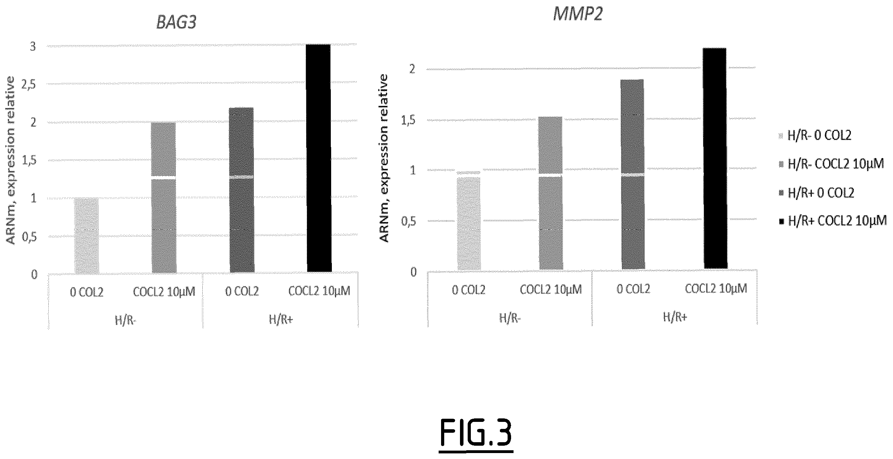

L'hypoxie/réoxygénation augmente l'expression de BAG3 et de MMP2 évaluée par RT-qPCR, autant que lors d'une incubation avec du chlorure de cobalt pendant une semaine. La réalisation d'un cycle d'hypoxie/réoxygénation en présence de chlorure de cobalt augmente d'autant plus l'expression de BAG3 et MMP2 (Figure 3).

En immunofluorescence, le signal de BAG3 est renforcé au niveau du domaine périnucléaire en présence de chlorure de cobalt. Le signal de BAG3 est retrouvé nucléaire lorsque l'on réalise un cycle d'hypoxie/réoxygénation en présence de chlorure de cobalt (Images non montrées).

Cette translocation nucléaire de BAG3, de la même manière que sur les zones les plus pathologiques des échantillons de patients, pourrait témoigner d'une susceptibilité accrue des cellules endothéliales à l'hypoxie/réoxygénation en présence d'un stress du réticulum endoplasmique préexistant.

### L'utilisation de M101 en présence de chlorure de cobalt ou d'hypoxie/réoxygénation rétablit l'expression de BAG3 et MMP2

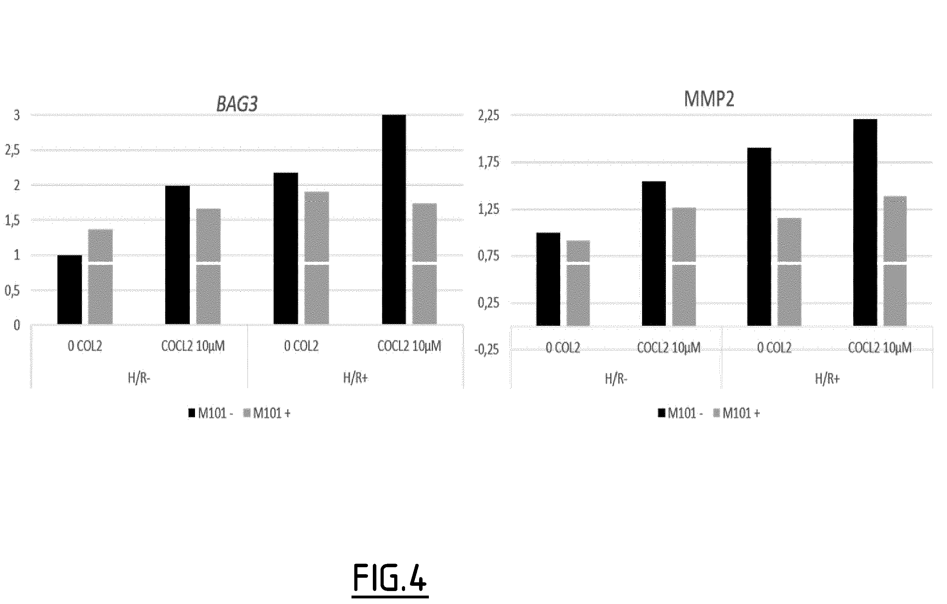

L'utilisation de l'hémoglobine M101 à une concentration de 0,5g/l diminue l'expression génique de *MMP2* et *BAG3* dans chaque condition de traitement. Cette diminution d'expression est plus marquée dans la condition hypoxie/réoxygénation en présence d'un stress du réticulum endoplasmique (Figure 4) généré par l'utilisation du chlorure de cobalt, que dans les conditions où l'on réalise un cycle d'hypoxie/réoxygénation seul ou celles où l'on incube les cellules avec du cobalt de manière isolée.

Le marquage en immunofluorescence de BAG3 ne met pas en évidence de signal nucléaire avec M101 (0,5g/L) dans la condition hypoxie/réoxygénation en présence de chlorure de cobalt (Images non montrées).

Ces résultats indiquent que l'utilisation de M101 sur le modèle *in vitro* semble reverser l'effet des fluctuations des niveaux d'oxygène dans les milieux de culture et pourrait protéger les cellules endothéliales en condition de stress hypoxique.

### Conclusion

L'ensemble des éléments présentés ci-dessus permettent d'avancer que l'hypoxie intermittente influe l'expression de marqueurs impliqués dans la physiopathologie de la dystrophie de Fuchs tels que BAG3 et MMP2. L'effet de l'hypoxie/réoxygénation est plus sévère en présence d'un stress du réticulum préexistant, stress cellulaire que l'on peut observer sur des échantillons de patients.

Ces données suggèrent que l'utilisation de l'hémoglobine *d'Arenicola marina* (M101) dans la dystrophie de Fuchs, sous différentes formes galéniques possibles, pourrait limiter les fluctuations d'oxygène nocturnes au niveau de l'endothélium cornéen. Son utilisation réduirait le stress cellulaire et *in fine* la perte cellulaire afin de ralentir l'évolution de la maladie vers un stade requérant une greffe de cornée, qui reste, à ce jour, le seul traitement curatif disponible.

### Références bibliographiques :

1. Bonanno JA. Identity and regulation of ion transport mechanisms in the corneal endothelium. Prog Retin Eye Res. janv 2003;22(1):69-94.
2. Eghrari AO, Riazuddin SA, Gottsch JD. Overview of the Cornea: Structure, Function, and Development. Prog Mol Biol Transl Sci. 2015;134:7-23.
3. Elbaz U, Mireskandari K, Tehrani N, Shen C, Khan MS, Williams S, et al. Corneal Endothelial Cell Density in Children: Normative Data From Birth to 5 Years Old. Am J Ophthalmol. janv 2017;173:134-8.
4. Gain P, Jullienne R, He Z, Aldossary M, Acquart S, Cognasse F, et al. Global Survey of Corneal Transplantation and Eye Banking. JAMA Ophthalmol. févr 2016;134(2):167-73.
5. Okumura N, Kitahara M, Okuda H, Hashimoto K, Ueda E, Nakahara M, et al. Sustained Activation of the Unfolded Protein Response Induces Cell Death in Fuchs' Endothelial Corneal Dystrophy. Invest Ophthalmol Vis Sci. 1 juill 2017;58(9):3697-707.
6. Xu I, Thériault M, Brunette I, Rochette PJ, Proulx S. Matrix metalloproteinases and their inhibitors in Fuchs endothelial corneal dystrophy. Exp Eye Res. 19 févr 2021;205:108500.
7. Zoega GM, Arnarsson A, Sasaki H, Söderberg PG, Jonasson F. The 7-year cumulative incidence of cornea guttata and morphological changes in the corneal endothelium in the Reykjavik Eye Study. Acta Ophthalmol. mai 2013;91(3):212-8.
8. Pang K, Lennikov A, Yang M. Hypoxia adaptation in the cornea: Current animal models and underlying mechanisms. Anim Models and Exp Med. déc 2021;4(4):300-10.

## Revendications

1. Molécule choisie parmi une globine d'Annélides, un protomère de globine d'Annélides et une hémoglobine extracellulaire d'Annélides, pour son utilisation pour le traitement de la dystrophie endothéliale cornéenne de Fuchs.

2. Molécule pour son utilisation selon la revendication 1, **caractérisée en ce que** l'hémoglobine extracellulaire d'Annélides est choisie parmi les hémoglobines extracellulaires d'Annélides Polychètes et les hémoglobines extracellulaires d'Annélides Oligochètes.

3. Molécule pour son utilisation selon la revendication 1 ou 2, **caractérisée en ce que** l'hémoglobine extracellulaire d'Annélides est choisie parmi les hémoglobines extracellulaires de la famille des *Lumbricidae,* les hémoglobines extracellulaires de la famille des *Arenicolidae* et les hémoglobines extracellulaires de la famille des *Nereididae,* de préférence parmi l'hémoglobine extracellulaire de *Lumbricus terrestris,* l'hémoglobine extracellulaire *d'Arenicola sp* et l'hémoglobine extracellulaire de *Nereis sp.*

4. Molécule pour son utilisation selon l'une des revendications précédentes, **caractérisée en ce que** l'hémoglobine extracellulaire d'Annélides est l'hémoglobine extracellulaire *d'Arenicola marina.*

5. Molécule pour son utilisation selon l'une des revendications précédentes, **caractérisée en ce qu'**elle est présente dans une composition en une teneur comprise entre 0,01% et 10% en poids par rapport au poids total de composition, de préférence entre 0,05% et 5% en poids, de préférence entre 0,06% et 2% en poids, de préférence entre 0,07% et 1% en poids.

6. Molécule pour son utilisation selon l'une des revendications précédentes, pour réduire significativement l'hypoxie intermittente des cellules endothéliales de la cornée.

7. Molécule pour son utilisation selon l'une des revendications précédentes, pour réduire le stress du réticulum endoplasmique des cellules endothéliales de la cornée.

8. Molécule pour son utilisation selon l'une des revendications précédentes, pour réduire significativement l'expression de *MMP2* et/ou de *BAG3* dans l'endothélium cornéen.

9. Molécule pour son utilisation selon l'une des revendications précédentes, pour protéger les cellules endothéliales cornéennes du stress hypoxique.

10. Molécule pour son utilisation selon l'une des revendications précédentes, **caractérisée en ce qu'**elle est formulée dans une composition pharmaceutique convenant pour une administration oculaire, de préférence choisie parmi les collyres, les pommades ophtalmiques, les gels ophtalmiques, les inserts conjonctivaux et les lentilles thérapeutiques.

## Patentansprüche

1. Molekül, ausgewählt aus einem Globin von Anneliden, einem Protomer von Globin von Anneliden und einem extrazellulären Hämoglobin von Anneliden zur Verwendung bei der Behandlung von Fuchs-Hornhaut-Endotheldystrophie.

2. Molekül zur Verwendung gemäß Anspruch 1, **dadurch gekennzeichnet, dass** das extrazelluläre Hämoglobin von Anneliden ausgewählt ist aus extrazellulären Hämoglobinen von Anneliden-Polychaeten und der Anneliden-Oligochaeten.

3. Molekül zur Verwendung nach Anspruch 1 oder 2, **dadurch gekennzeichnet, dass** das extrazelluläre Hämoglobin von Anneliden ausgewählt ist aus extrazellulären Hämoglobinen der Familien *Lumbricidae,* extrazellulären Hämoglobinen der Familie *Arenicolidae* und extrazellulären Hämoglobinen der Familie *Nereididae,* vorzugsweise aus dem extrazellulären Hämoglobin von *Lumbricus terrestris,* dem extrazellulären Hämoglobin von *Arenicola sp* und dem extrazellulären Hämoglobin von *Nereis sp.*

4. Molekül zur Verwendung nach einem der vorherigen Ansprüche, **dadurch gekennzeichnet, dass** das extrazelluläre Hämoglobin von Anneliden das Hämoglobin von *Arenicola marina* ist.

5. Molekül zur Verwendung nach einem der vorherigen Ansprüche, **dadurch gekennzeichnet, dass** es in einer Zusammensetzung mit einem Gehalt zwischen 0,01 und 10 Gewichts-% bezogen auf das Gesamtgewicht der Zusammensetzung, vorzugsweise zwischen 0,05 und 5 Gewichts-%, vorzugsweise zwischen 0,06 und 2 Gewichts-% und vorzugsweise zwischen 0,07 und 1 Gewichts-% vorhanden ist.

6. Molekül zur Verwendung nach einem der vorherigen Ansprüche, um die intermittierende Hypoxie von Endothelzellen der Hornhaut signifikant zu reduzieren.

7. Molekül zur Verwendung nach einem der vorherigen Ansprüche, um den Stress des endoplasmatischen Retikulums der Endothelzellen der Hornhaut zu reduzieren.

8. Molekül zur Verwendung nach einem der vorherigen Ansprüche, um die Expression von *MMP2* und/oder *BAG3* in dem Hornhaut-Endothel signifikant zu reduzieren.

9. Molekül zur Verwendung nach einem der vorherigen Ansprüche, um Hornhaut-Endothelzellen vor hypoxischem Stress zu schützen.

10. Molekül zur Verwendung nach einem der vorherigen Ansprüche, **dadurch gekennzeichnet, dass** es in einer pharmazeutischen Zusammensetzung formuliert ist, die für eine okuläre Verabreichung geeignet ist, vorzugsweise ausgewählt aus Augentropfen, Augensalben, Augengele, konjunktivalen Inserts und therapeutischen Linsen.

## Claims

1. A molecule selected from Annelida globin, Annelida globin protomer and Annelida extracellular hemoglobin for use in the treatment of Fuchs' corneal endothelial dystrophy.

2. The molecule for use according to claim 1, **characterized in that** the Annelid extracellular hemoglobin is chosen from Polychaete Annelid extracellular hemoglobins and Oligochaete Annelid extracellular hemoglobins.

3. The molecule for use according to claim 1 or 2, **characterized in that** the Annelid extracellular hemoglobin is chosen from extracellular hemoglobins of the family *Lumbricidae,* extracellular hemoglobins of the family *Arenicolidae* and extracellular hemoglobins of the family *Nereididae,* preferably from *Lumbricus terrestris* extracellular hemoglobin, *Arenicola sp* extracellular hemoglobin and *Nereis sp* extracellular hemoglobin.

4. The molecule for use according to one of the preceding claims, **characterized in that** the Annelid extracellular hemoglobin is the *Arenicola marina* extracellular hemoglobin

5. The molecule for use according to one of the preceding claims, **characterized in that** it is present in a composition in a content comprised between 0.01% and 10% by weight relative to the total weight of the composition, preferably between 0.05% and 5% by weight, preferably between 0.06% and 2% by weight, preferably between 0.07% and 1% by weight.

6. The molecule for use according to any of the preceding claims, for significantly reducing intermittent hypoxia of corneal endothelial cells.

7. The molecule for use according to one of the preceding claims, for reducing the stress of the endoplasmic reticulum of corneal endothelial cells.

8. The molecule for use according to one of the preceding claims, for significantly reducing the expression of *MMP2* and/or BAG3 in the corneal endothelium.

9. The molecule for use according to one of the preceding claims, for protecting corneal endothelial cells from hypoxic stress.

10. The molecule for use according to one of the preceding claims, **characterized in that** it is formulated in a pharmaceutical composition suitable for ocular administration, preferably chosen from eye drops, ophthalmic ointments, ophthalmic gels, conjunctival inserts and therapeutic lenses.
